# EUROPÄISCHE PATENTANMELDUNG

(11) **EP 2 255 734 A1**
(43) Veröffentlichungstag der Anmeldung: **01.12.2010**
(21) Anmeldenummer: 10160606.9
(22) Anmeldetag: 21.04.2010
(51) Int. Cl.: A61B 17/32, F16C 1/04

(54) **Chirurgisches Instrument**

(30) Priorität: 29.05.2009 DE 102009024244; 05.08.2009 DE 102009037153
(71) Anmelder: AESCULAP AG, 78532 Tuttlingen (DE)
(72) Erfinder: Hermann, Reiner, 78567, Fridingen (DE)
(74) Vertreter: Hoeger, Stellrecht & Partner Patentanwälte

(57) **Zusammenfassung**

Es wird ein chirurgisches Instrument mit einem proximalen und einem distalen Endabschnitt, umfassend einen lang gestreckten, hohlen, sich vom proximalen zum distalen Endabschnitt erstreckenden, äußeren Schaft, ein in dem äußeren Schaft drehbar gelagertes, hohlzylindrisches Antriebselement sowie ein am distalen Endabschnitt des Instruments angeordnetes, mit dem Antriebselement gekoppeltes Schneid-, Abrasiv- oder Fräswerkzeug, vorgeschlagen, wobei das Antriebselement einen zwischen dem proximalen und distalen Endabschnitt angeordneten flexiblen Abschnitt aufweist, der eine Mehrzahl an Ringsegmenten umfasst, welche in Axialrichtung jeweils einen ersten und einen zweiten Endbereich aufweisen, wobei der erste Endbereich zwei oder mehr in Axialrichtung abstehende Vorsprünge und der zweite Endbereich zwei oder mehr die Vorsprünge aufnehmende Rücksprünge umfasst und die Ringsegmente über die Vor- und Rücksprünge gelenkig ineinander greifen, **dadurch gekennzeichnet, dass** die Ringsegmente über die Vor- und Rücksprünge in Axial- und/oder Radialrichtung miteinander formschlüssig verbunden sind.

Das erfindungsgemäße chirurgische Instrument ermöglicht bei minimalem Aufwand in der Herstellung einen sicheren Betrieb, insbesondere auch bei hohen Drehzahlen.

## Beschreibung

Die Erfindung betrifft ein chirurgisches Instrument für minimalinvasive chirurgische Eingriffe mit einem an einem so genannten distalen Ende eines Schafts an ein Antriebselement gekoppelten Werkzeug. Solche Instrumente werden häufig auch Shaver genannt.

Chirurgische Instrumente dieser Art weisen einen proximalen und einen distalen Endabschnitt auf, einen lang gestreckten hohlen äußeren Schaft, ein in dem äußeren Schaft drehbar gelagertes, hohlzylindrisches Antriebselement sowie ein am distalen Endabschnitt des Instruments angeordnetes, mit dem Antriebselement gekoppeltes Schneid-, Abrasiv- oder Fräswerkzeug.

Neben geradlinig ausgebildeten Instrumenten sind auch chirurgische Instrumente bekannt, bei denen der distale Endbereich abgewinkelt oder gekröpft ist, um mit dem chirurgischen Instrument auch schwieriger zugängliche Arbeitspositionen erreichen zu können und um den Arbeitsbereich des Instruments generell zu vergrößern.

Es ist in diesem Zusammenhang bekannt, das Antriebselement zwischen dem proximalen und distalen Endabschnitt mit einem flexiblen Abschnitt auszustatten, der eine Mehrzahl an Ringsegmenten umfasst, welche in Axialrichtung jeweils einen ersten und einen zweiten Endbereich aufweisen, wobei der erste Endbereich zwei oder mehr in Axialrichtung abstehende Vorsprünge und der zweite Endbereich zwei oder mehr die Vorsprünge aufnehmende Rücksprünge umfasst und die Ringsegmente über die Vor- und Rücksprünge gelenkig ineinander greifen.

Chirurgische Instrumente dieser Art sind beispielsweise aus der EP 0 677 276 B1 bekannt, wobei der flexible Abschnitt erlaubt, das Drehmoment vom proximalen Endabschnitt des Antriebselements wirksam auf den distalen Endabschnitt und damit auf das dort angeschlossene Werkzeug zu übertragen.

Da der Shaver typischerweise zur Entfernung von Körpergewebe dient, wird der sich im Inneren des Schafts und des Antriebselements vorhandene Kanal zum Absaugen der entfernten Gewebeteile verwendet.

Nachteilig bei diesen bekannten Instrumenten ist, dass die Herstellung, insbesondere die Montage des flexiblen Abschnittes aufwändig ist, da der flexible Abschnitt aus einer Vielzahl von Ringsegmenten nur lose zusammengesetzt ist, sodass nicht nur bei der Herstellung, sondern auch bei einer späteren Demontage des Antriebselements und dessen Entnahme aus dem hohlzylindrischen äußeren Schaft, Ringsegmente verloren gehen können, insbesondere auch im OP.

Ein weiterer Shaver, der diesem Prinzip der Konstruktion des Antriebselements mit im flexiblen Abschnitt lose ineinander greifenden Ringsegmenten folgt, ist aus der DE 10 2004 046 539 A1 bekannt.

Ein anderer Ansatzpunkt wurde in der EP 0 986 989 B1 gewählt, bei dem der flexible Abschnitt durch ein hohlzylindrisches Element gebildet wird, dessen Wandung schraubenlinienförmig in Radialrichtung geschlitzt ist, wobei sich mäanderförmig abwechselnd Zähne und Einbuchtungen entlang der Schraubenlinie abwechseln und ineinander greifen, so dass in Axialrichtung ein Zusammenhalt der Windungen gegeben ist.

Nachteilig bei dieser Lösung ist eine erheblich geringere Flexibilität und häufig vorkommende Ermüdungsbrüche, die durch die Wechselbiegebeanspruchung beim Drehen der Welle auftreten. Ein oszillierender Antrieb soll mit diesem Instrument zwar möglich sein, jedoch ist es mit hohen Drehzahlen nur in einer Drehrichtung antreibbar.

Aufgabe der Erfindung ist es, ein chirurgisches Instrument der eingangs beschriebenen Art so weiter zu bilden, dass bei minimalem Aufwand in der Herstellung ein sicherer Betrieb, insbesondere auch bei hohen Drehzahlen möglich ist.

Diese Aufgabe wird erfindungsgemäß bei einem chirurgischen Instrument dadurch gelöst, dass die Ringsegmente über die Vor- und Rücksprünge in Axial-und/oder Radialrichtung miteinander formschlüssig verbunden sind.

Durch den Formschluss der Vor- und Rücksprünge in Axial- und/oder Radialrichtung, vorzugsweise in Axial- und Radialrichtung, wird die Handhabung des Antriebselements bei der Herstellung, beim Zusammenbau sowie bei einer späteren Demontage erheblich erleichtert. Die gelenkige Verbindung der Ringsegmente im flexiblen Abschnitt ist trotzdem gewährleistet.

Werden die Ringsegmente mit mehr als zwei Vor- und Rücksprüngen versehen, lassen sich pro Ringsegmentpaar mehrere Gelenkachsen realisieren.

Der Formschluss in Axial- oder Radialrichtung vermindert in erheblichem Umfang das Risiko einzelne Ringsegmente zu verlieren, wenn der Verlust nicht durch den bevorzugt axial und radial gegebenen Formschluss von vornherein ausgeschlossen wird.

Über den Formschluss in Axial- und/oder Radialrichtung kann auch die gegenseitige Positionierung der aneinandergrenzenden Ringsegmente so genau vorgegeben werden, dass hohe Drehzahlen auch in beiden möglichen Antriebsrichtungen realisiert werden können.

Der Formschluss in Axialrichtung lässt sich durch die Verwendung von mehr als zwei Vor- und Rücksprüngen verbessern.

Typischerweise werden die Vor- und Rücksprünge in regelmäßigen Abständen in Umfangsrichtung am jeweiligen Ringsegment angeordnet. Dies gewährleistet eine gleichmäßige Belastung der Ringsegmente bei der Übertragung der Antriebskräfte und damit eine möglichst große Lebensdauer.

Gemäß einer Ausführungsform der vorliegenden Erfindung ist die Zahl der Vorsprünge und Rücksprünge ungerade gewählt, woraus sich insbesondere bei einer Anordnung der Vor- und Rücksprünge in regelmäßigen Abständen in Umfangsrichtung am jeweiligen Ringsegment ein Formschluss in Radialrichtung ohne weitere Maßnahmen ergibt.

Zusätzlich oder alternativ kann der Formschluss in Radialrichtung dadurch erreicht werden, dass die Vorsprünge an ihrer radial außen liegenden Seite in Umfangsrichtung eine größere Ausdehnung aufweisen als auf ihrer radial innen liegenden Seite und dass die Rücksprünge entsprechend auf ihrer radial innen liegenden Seite in Umfangsrichtung eine Ausdehnung aufweisen, die kleiner ist als die entsprechende radial außen liegende Ausdehnung im entsprechenden Bereich des in den Rücksprung eingreifenden Vorsprungs.

Mit dieser Maßnahme kann ein Formschluss in Radialrichtung erzielt werden, unabhängig davon, ob die Zahl der Vor- und Rücksprünge ungerade oder gerade ist.

Der Formschluss in Axialrichtung kann dadurch erreicht werden, dass die Vorsprünge an ihrem dem Ringsegment abgewandten, freien Ende eine größere Ausdehnung in Umfangsrichtung aufweisen als an ihrem zum Ringsegment benachbarten Ende und die Rücksprünge an ihrem offenen Ende eine kleinere Ausdehnung in Umfangsrichtung aufweisen als die Ausdehnung des in den Rücksprung eingreifenden Vorsprungs an seinem freien Ende in Umfangsrichtung.

Wird ein Formschluss sowohl in Axialrichtung als auch in Radialrichtung verwirklicht, hat man ein Antriebselement vorliegen, das als Ganzes gehandhabt werden kann ohne die Gefahr, dass einzelne Teile des flexiblen Abschnitts verloren gehen.

Vorzugsweise wird ein solches Antriebselement mit einem flexiblen Abschnitt so hergestellt, dass zunächst ein einstückiges hohlzylindrisches Element verwendet wird, bei dem beispielsweise mit Laser die Konturen der Vor- und Rücksprünge eingeschnitten werden. Häufig reicht der dabei entstehende Schneidspalt bereits aus, um eine ausreichende Verschwenkung der einzelnen Ringsegmente gegeneinander sicherzustellen, sodass der flexible Abschnitt des Antriebselements einen für die jeweilige Anwendung ausreichenden Biegewinkel bereitstellen kann.

Ist in Axialrichtung bereits ein Formschluss vorhanden, so kann der Formschluss in Radialrichtung auch dadurch erzielt werden, dass die Vorsprünge in Axialrichtung auf der radial außen liegenden Seite eine größere Ausdehnung in Längsrichtung aufweisen als auf der radial innen liegenden Seite und die Rücksprünge auf der radial innen liegenden Seite eine Tiefe in Axialrichtung aufweisen, die geringer ist als die Länge der Vorsprünge in Axialrichtung auf deren außen liegenden Seite.

Bei einer bevorzugten Ausführungsform der Erfindung werden in Umfangsrichtung Anlageflächen auf Seiten der Vorsprünge als auch korrespondierend bei den Rücksprüngen vorgesehen, die eben sind. Damit lässt sich die Drehmomentübertragung so optimieren, dass ein geringerer Verschleiß und die Möglichkeit, sehr hohe Drehzahlen zuzulassen, resultieren.

Eine bevorzugte Form der Vorsprünge in Umfangsrichtung ist die Trapezform, auch wenn selbstverständlich hiervon abweichende Formen die Realisierung eines Formschlusses in Axialrichtung ermöglichen. Korrespondierend hierzu sind die Rücksprünge in Umfangsrichtung vorzugsweise trapezförmig ausgebildet. Hier ergeben sich dann großflächige ebene Anlageflächen der Vor- und Rücksprünge.

Für die Sicherstellung eines Formschlusses in Radialrichtung sind die Vorsprünge vorzugsweise mit einem in der Radialrichtung gesehenen trapezförmigen Querschnitt ausgebildet, wobei hier die Wölbungen an der radial außen liegenden und radial innen liegenden Oberfläche der Vorsprünge bei dieser Definition unberücksichtigt bleiben.

Korrespondierend hierzu weisen die Rücksprünge in Radialrichtung ebenfalls vorzugsweise einen trapezförmigen Querschnitt auf.

Diese und weitere Vorteile der Erfindung werden im Folgenden anhand der Zeichnung noch näher erläutert. Es zeigen im Einzelnen:
- Figur 1: eine perspektivische Darstellung eines erfindungsgemäßen chirurgi- schen Instruments in Form eines Shavers;
- Figur 2: einen vergrößerten Teilbereich des Shavers aus Figur 1;
- Figur 3: eine alternative Ausführungsform des distalen Endes der Figur 2;
- Figur 4: eine Einzelheit des flexiblen Antriebselements;
- Figur 5: eine alternative Ausführungsform eines Teils des flexiblen Antriebs- elements; und
- Figur 6: eine schematische Darstellung der Art der Verwendung des erfin- dungsgemäßen chirurgischen Instruments.

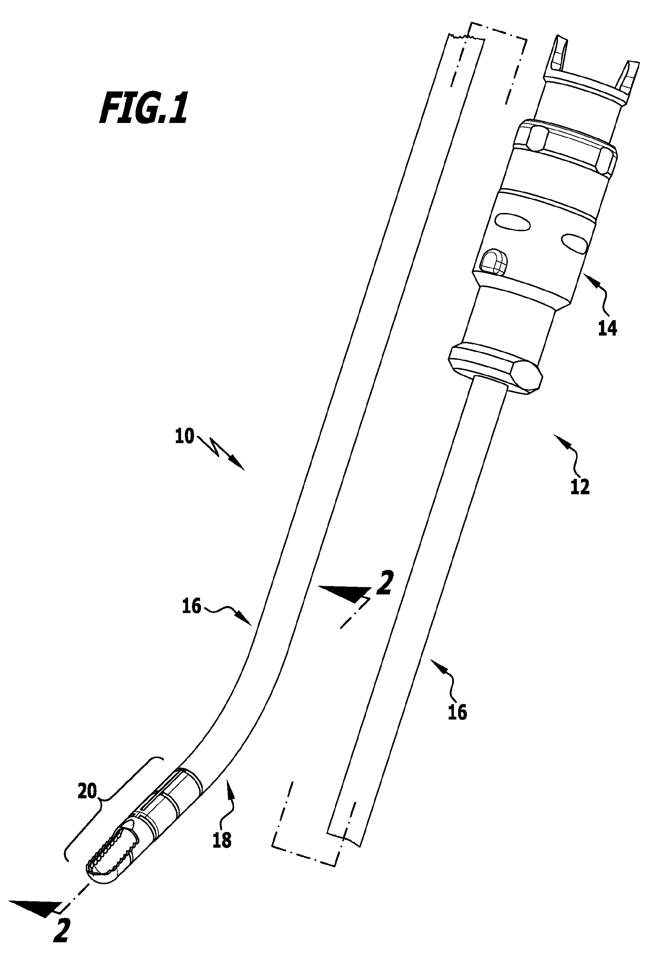

Figur 1 zeigt ein insgesamt mit dem Bezugszeichen 10 bezeichnetes chirurgisches Instrument gemäß der vorliegenden Erfindung, das im Folgenden auch kurz Shaver genannt wird.

Der erfindungsgemäße Shaver 10 weist an seinem proximalen Ende 12 eine Kupplungsvorrichtung 14 auf, welches mit einem chirurgischen Antrieb (nicht dargestellt) verbunden werden kann.

Distalseitig schließt sich an die Kupplungsvorrichtung 14 ein biege- und torsionsfester Schaft 16 an, der im Bereich seines distalen Endabschnitts 18 um etwa 20° gegenüber der Längsachse des Shavers 10 geneigt ist und einen distalen Schaftabschnitt 20 umfasst.

Die Kupplungsvorrichtung 14 ist bereits in der DE 10 2004 046 539 A1 in Einzelheiten gezeigt und beschrieben, sodass auf diese Beschreibung dort wegen der Einzelheiten verwiesen werden kann.

Der Schaft 16 mit seinem distalen Endabschnitt 20 ist als lang gestreckte äußere Hülse 22, in dem ein hohlzylindrisches Antriebselement 24 geführt ist, ausgebildet. Der Schaft 16 bzw. die Hülse 22 ist an dem distalen Ende geschlossen und mit einer seitlichen Öffnung 26 versehen, welche einen Durchlass vom Innenraum der Hülse 22 zu der Umgebung des Shavers 10 schafft.

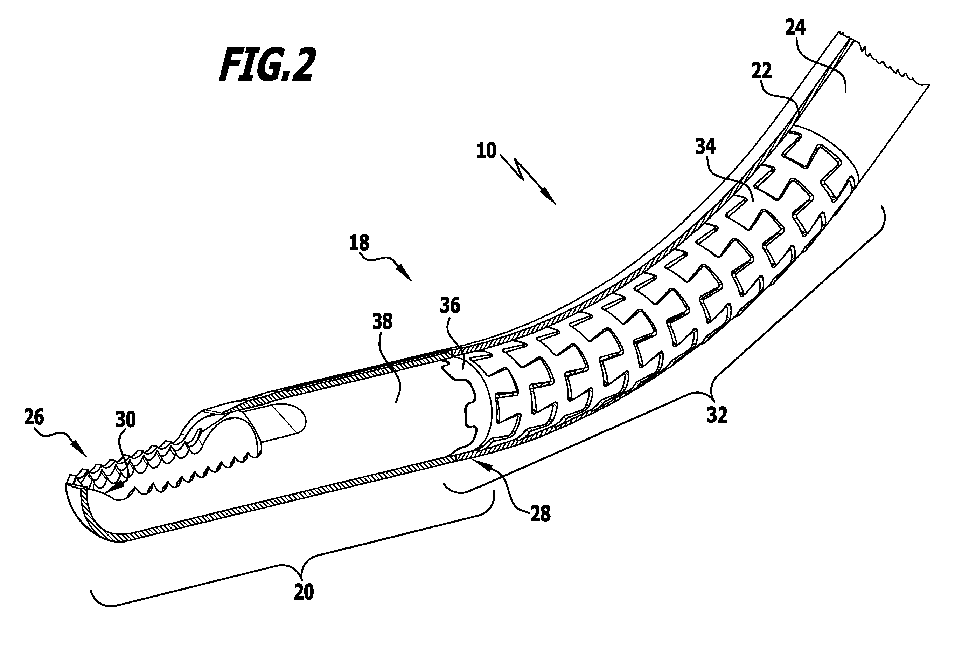

An das Antriebselement 24 ist an seinem distalen Ende 28 ein Werkzeug 38 angekoppelt, das ebenfalls eine seitliche Öffnung 30 aufweist, welche im Wesentlichen korrespondierend zu der seitlichen Öffnung 26 der Hülse 22 ausgebildet ist. Die Öffnungen 26 und 30 sind, wie aus Figur 2 ersichtlich, an ihren Rändern angeschliffen und mit Schneiden versehen. Wird das Antriebselement 24 in Rotation versetzt, so werden in den Bereich der seitlichen Öffnungen 26, 30 gelangende Gewebeteile durch die Schneiden der Ränder der Öffnungen 26, 30 abgetrennt und können über den im Antriebelement 24 verbleibenden Innenraum mittels Unterdruck abgesaugt werden.

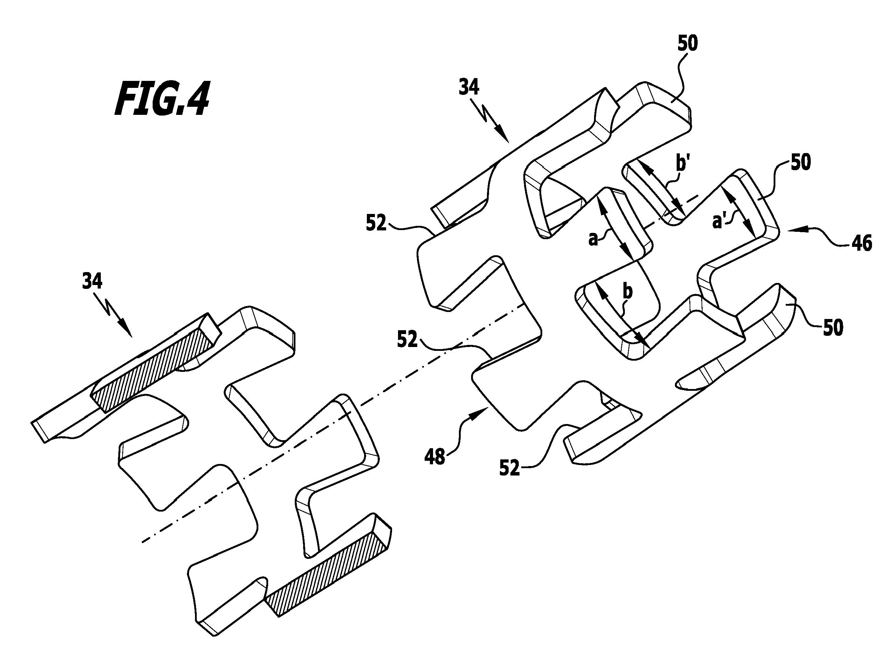

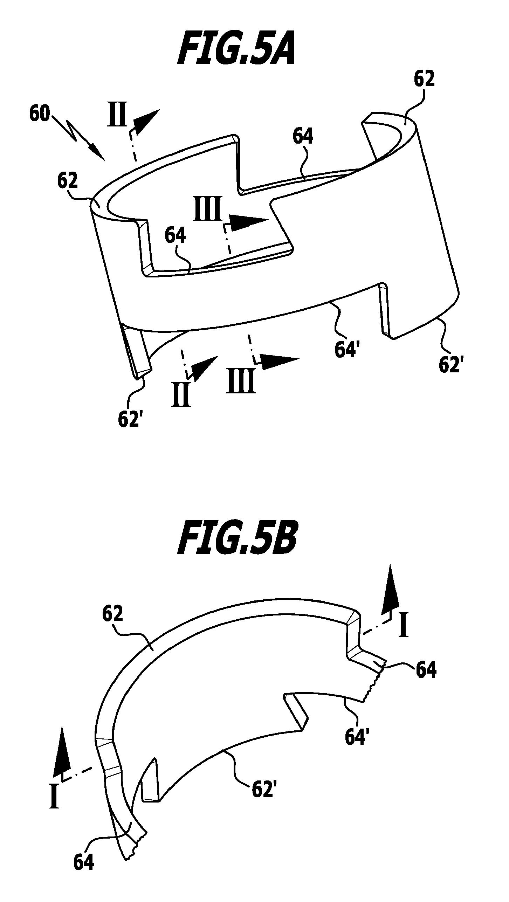

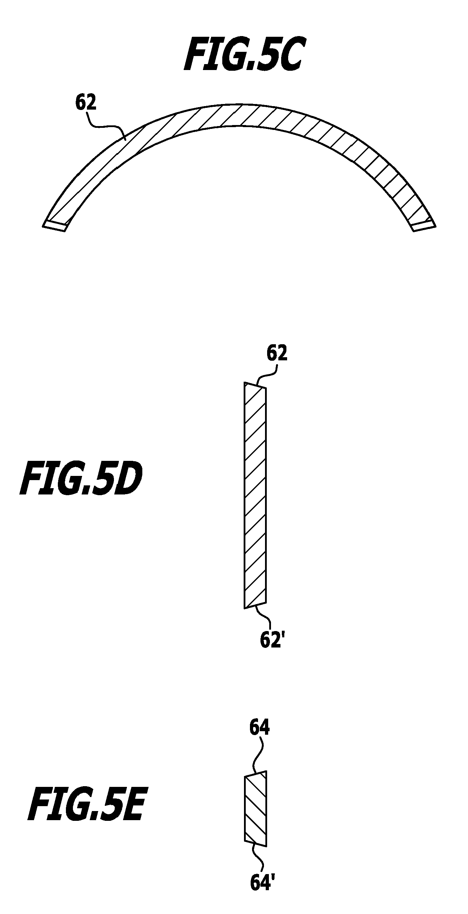

Im Endabschnitt 18 des Shavers 10 geht das hohlzylindrische Antriebselement 24 aus einem einstückigen oder massiven hohlzylindrischen Teil in einen hohlzylindrischen flexiblen Abschnitt 32 über, der von einer Anzahl an Ringsegmenten 34 gebildet wird, die im Folgenden anhand der Figuren 4 und 5 noch näher beschrieben werden.

Das distale Ende des Antriebselements 24 endet mit einem Kupplungsstück 36, an das lose eingreifend ein Werkzeug, insbesondere das Fräswerkzeug 38, angekoppelt werden kann.

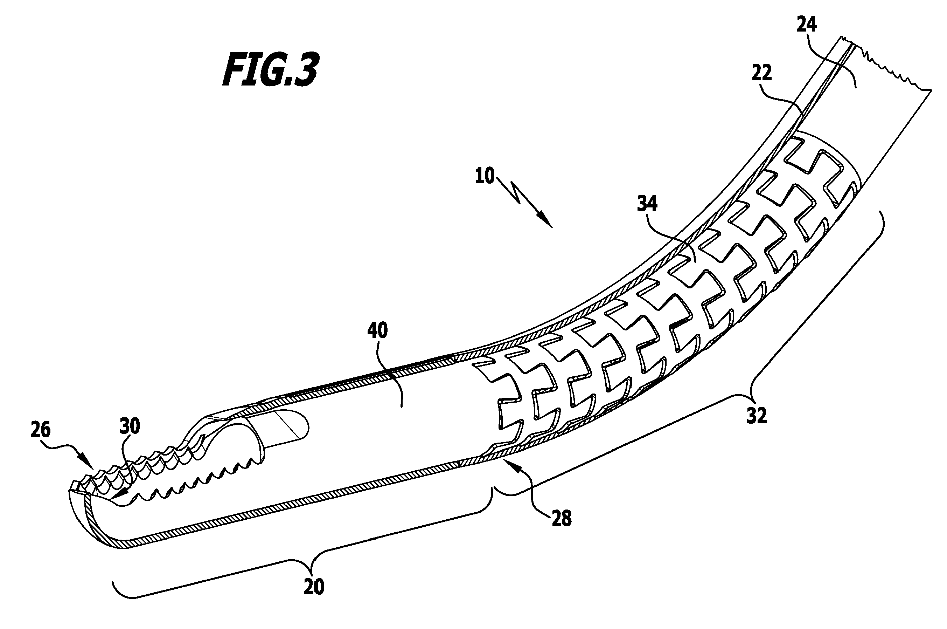

Figur 3 zeigt wiederum das distale Ende 20 des Shavers 10 in einer alternativen Ausführungsform, bei der anstelle des über ein Kupplungsstück 36 angekoppelten Werkzeugs 38 ein Werkzeug 40 unverlierbar im Formschluss gehalten ist.

Unterschiede ergeben sich bei diesen beiden Ausführungsformen in der Fertigung der Instrumente:
Bei der Ausführungsform der Figur 2 wird beim Zusammenbau zunächst das Werkzeug 38 in die Hülse 22 eingeführt, wobei dann der distale Endabschnitt gebogen und in seine endgültige Gestalt gebracht wird. Danach lässt sich das Antriebselement 24 mit seinem flexiblen Abschnitt 32 in dies Hülse einsetzen, wobei das Kupplungsstück 36 in proximale Ende des Werkzeugs 38 eingreift.

Bei der Ausführungsform des Figur 3 wird dagegen das Antriebselement 24 zusammen hängend mit dem Werkzeug 40 gefertigt und in die Hülse 22 eingeschoben, solange diese noch eine gerade Form aufweist. Erst danach wird die Hülse 22 gebogen und in ihre endgültige, gekröpfte Form gebracht.

Die Ringsegmente 34 sind in Figur 4 im Einzelnen dargestellt und weisen einen ersten Endbereich 46 in einer ersten Axialrichtung auf und einen zweiten Endbereich 48 in der entgegengesetzten Axialrichtung. An seinem ersten Endbereich weist das Ringsegment 34 sechs Vorsprünge 50 auf, die regelmäßig in Umfangsrichtung verteilt an dem Ringsegment 34 angeordnet sind. An seinem axial entgegengesetzten Endbereich 48 weist das Ringsegment 34, korrespondierend zu den Vorsprüngen 50 geformte Ausnehmungen oder Rücksprünge 52 auf, wobei die Vorsprünge und Rücksprünge in Umfangsrichtung gesehen jeweils eine Trapezform aufweisen, wobei bei den Vorsprüngen 50 das in Axialrichtung freie Ende der Vorsprünge 50 eine größere Ausdehnung in Umfangsrichtung aufweist als das am Ringsegment benachbart liegende Ende, während korrespondierend die Ausnehmungen 52 an ihrem inneren Ende eine größere Ausdehnung in Umfangsrichtung aufweisen als die in Richtung zum Endbereich 48 liegenden nach außen offenen Abschnitte. Im Fall der Ringsegmente 34, die in Figur 4 im Detail gezeigt sind, sind aufgrund der regelmäßigen Geometrie beide Endbereich mit einer bis auf die spiegelbildliche Anordnung identischen Ausgestaltung versehen.

Dadurch, dass Vorsprünge 50 in korrespondierend geformte Rücksprünge 52 mit Spiel und trotzdem formschlüssig eingreifen, ist ein axialer Formschluss gegeben, sodass die Ringsegmente 34 in Axialrichtung miteinander verbunden und so zusammen in die äußere Hülse 22 eingeführt bzw. aus dieser herausgezogen werden können.

Trotzdem bleibt aufgrund der bestehenden Spalte zwischen den Vorsprüngen 50 und den Rücksprüngen 52 ausreichend Spiel um eine gelenkige Verbindung benachbarter Ringsegmente 34 zu gewährleisten.

Die Vorsprünge 50 und die Ausnehmungen 52 weisen jeweils miteinander in Kontakt kommende Anlageflächen 54, 56 auf, die bei dem in Figur 5 gezeigten Ausführungsbeispiel großflächig eben ausgebildet sind. Damit ist eine optimale Drehmomentübertragung zwischen den einzelnen, aufeinander folgenden Ringsegmenten möglich.

Aufgrund der Vielzahl an Vor- und Rücksprüngen 50, 52 ergeben sich mehrere Schwenkachsen für die Verbindung zweier aufeinander folgender Ringsegmente, so dass das Antriebselement 24 dem gekrümmten Verlauf des hohlzylindrischen Schafts 16 genau folgen kann.

Durch die Vielzahl an ineinander greifenden Vor- und Rücksprüngen 50, 52 mit trapezförmiger Konfiguration ist eine besonders sichere Verbindung in Axialrichtung gegeben.

Um das Antriebelement 24 mit seinem flexiblen Abschnitt 32 und den diesen bildenden Ringsegmenten 34 insgesamt problemlos handhaben zu können, ist vorgesehen, dass die Ausdehnung der Vorsprünge 50 an ihrem freien Ende in Umfangsrichtung (a) größer ist als deren korrespondierende Ausdehnung auf der Innenseite (a').

Entsprechend sind die Ausnehmungen 52 an ihrem äußeren und inneren Umfang mit einer Breite b bzw. einer Breite b' dimensioniert, die auch in Radialrichtung einen Formschluss ermöglichen, sodass die Ringsegmente 34 unverlierbar miteinander verbunden und handhabbar sind.

Während bei den Ringsegmenten der Figur 4 jeweils sechs Vorsprünge und sechs Rücksprünge vorgesehen sind, sind bei einer alternativen Ausführungsform der Ringsegmente 60 in Figur 5, die die einfachste Ausführungsform zeigen, jeweils nur zwei Vorsprünge 62 und zwei Rücksprünge 64 an den axial entgegengesetzten Enden des Ringsegments 60 vorhanden, die jedoch in ähnlicher Weise bei der Realisierung der vorliegenden Erfindung Anwendung finden können. Lediglich der realisierbare Schwenkwinkel zweier aneinandergrenzender Ringsegmente 60 ist gegenüber zwei aneinandergrenzenden Ringsegmente 34 etwas eingeschränkt.

Auch hier gilt, dass die Vor- und Rücksprünge 62, 64 in Axial- und/oder Radialrichtung miteinander formschlüssig verbunden sein sollen, sodass auf die Ausführungen zur Figur 4 betreffend die Ausgestaltung der Vorsprünge 50 bzw. der Rücksprünge 52 Bezug genommen werden kann.

Die Schnittdarstellungen der Figuren 5b bis 5e verdeutlichen die trapezförmigen Querschnitte der Vorsprünge 62 und der Rücksprünge 64 in Umfangs- wie auch in Axialrichtung. Dies gilt auch für die Ausbildung der Vor- und Rücksprünge des Ringsegments 34.

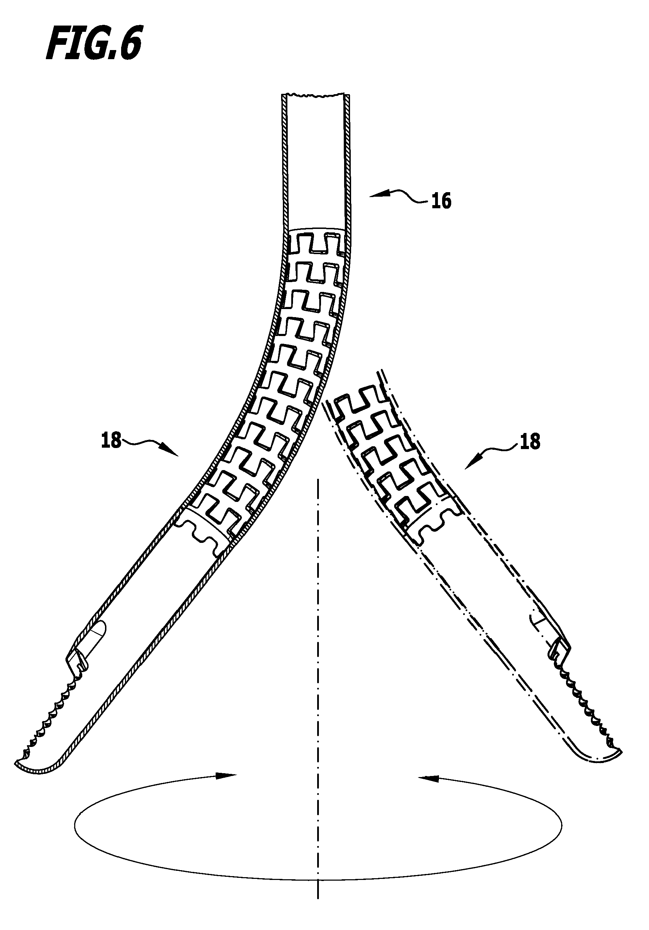

In der Übersichtsfigur 6 ist schließlich angedeutet, welche Bedeutung der abgewinkelte Bereich im distalen Endabschnitt 18 des Schafts 16 für den mit dem Shaver erreichbaren Arbeitsbereich aufweist, der durch die Abwinkelung wie in Figur 6 dargestellt erheblich größer ausfällt als bei einem nur geradlinig geführten Schaft.

## Patentansprüche

1. Chirurgisches Instrument mit einem proximalen und einem distalen Endabschnitt, umfassend einen lang gestreckten, hohlen, sich vom proximalen zum distalen Endabschnitt erstreckenden, äußeren Schaft, ein in dem äußeren Schaft drehbar gelagertes, hohlzylindrisches Antriebselement sowie ein am distalen Endabschnitt des Instruments angeordnetes, mit dem Antriebselement gekoppeltes Schneid-, Abrasiv- oder Fräswerkzeug,
wobei das Antriebselement einen zwischen dem proximalen und distalen Endabschnitt angeordneten flexiblen Abschnitt aufweist, der eine Mehrzahl an Ringsegmenten umfasst, welche in Axialrichtung jeweils einen ersten und einen zweiten Endbereich aufweisen, wobei der erste Endbereich zwei oder mehr in Axialrichtung abstehende Vorsprünge und der zweite Endbereich zwei oder mehr die Vorsprünge aufnehmende Rücksprünge umfasst und die Ringsegmente über die Vor- und Rücksprünge gelenkig ineinander greifen, **dadurch gekennzeichnet, dass** die Ringsegmente über die Vor-und Rücksprünge in Axial- und/oder Radialrichtung miteinander formschlüssig verbunden sind.

2. Instrument nach Anspruch 1, **dadurch gekennzeichnet, dass** die Vor- und Rücksprünge in regelmäßigen Abständen in Umfangsrichtung am jeweiligen Ringsegment angeordnet sind.

3. Instrument nach Anspruch 1 oder 2, **dadurch gekennzeichnet, dass** die Zahl der Vorsprünge und Rücksprünge ungerade ist.

4. Instrument nach einem der Ansprüche 1 bis 3, **dadurch gekennzeichnet, dass** die Vorsprünge an ihrer radial außen liegenden Seite in Umfangsrichtung eine größere Ausdehnung aufweisen als auf ihrer radial innen liegenden Seite und dass die Rücksprünge auf der radial innen liegenden Seite in Umfangsrichtung eine Ausdehnung aufweisen, die kleiner ist als die entsprechende radial außen liegende Ausdehnung des in den Rücksprung eingreifenden Vorsprungs.

5. Instrument nach einem der Ansprüche 1 bis 4, **dadurch gekennzeichnet, dass** die Vorsprünge an ihrem dem Ringsegment abgewandten freien Ende in Umfangsrichtung eine größere Ausdehnung aufweisen als benachbart zum Ringsegment und dass die Rücksprünge an ihrem offenen Ende eine kleinere Ausdehnung in Umfangsrichtung aufweisen als die Ausdehnung des in den Rücksprung eingreifenden Vorsprungs an seinem freien Ende.

6. Instrument nach einem der Ansprüche 1 bis 5, **dadurch gekennzeichnet, dass** die Vorsprünge in Axialrichtung auf der radial außen liegenden Seite eine größere Ausdehnung aufweisen als auf der radial innen liegenden Seite und dass die Rücksprünge auf der radial innen liegenden Seite eine Tiefe in Axialrichtung aufweisen, die geringer ist als die Länge der Vorsprünge in Axialrichtung auf deren außen liegenden Seite.

7. Instrument nach einem der Ansprüche 1 bis 6, **dadurch gekennzeichnet, dass** die Vorsprünge in Umfangsrichtung trapezförmig ausgebildet sind.

8. Instrument nach einem der Ansprüche 1 bis 7, **dadurch gekennzeichnet, dass** die Rücksprünge in Umfangsrichtung trapezförmig ausgebildet sind.

9. Instrument nach einem der Ansprüche 1 bis 8, **dadurch gekennzeichnet, dass** die Vorsprünge in Radialrichtung einen trapezförmigen Querschnitt aufweisen.

10. Instrument nach einem der Ansprüche 1 bis 9, **dadurch gekennzeichnet, dass** die Rücksprünge in Radialrichtung einen trapezförmigen Querschnitt aufweisen.

11. Instrument nach einem der Ansprüche 1 bis 10, **dadurch gekennzeichnet, dass** die Vorsprünge und Rücksprünge in Umfangsrichtung miteinander in Kontakt tretende ebene Anlageflächen aufweisen.
